(19)

Europäisches
Patentamt
European
Patent Office
Office européen
des brevets

(11) **EP 3 395 335 A1**

(12) **EUROPEAN PATENT APPLICATION**

(43) Date of publication:
**31.10.2018 Bulletin 2018/44**

(51) Int Cl.:
***A61K 9/70*** *(2006.01)*     ***A61K 31/565*** *(2006.01)*

(21) Application number: **17382218.0**

(22) Date of filing: **24.04.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Kern Pharma, S.L.
08228 Terrassa (ES)**

(72) Inventors:
- **Soler Ranzani, Luis
  08013 BARCELONA (ES)**
- **Sallán Leyes, Marta
  08191 RUBÍ (ES)**
- **Millán Martínez, David
  08224 TERRASSA (ES)**

(74) Representative: **ZBM Patents - Zea, Barlocci &
Markvardsen
Plaza Catalunya, 1 2nd floor
08002 Barcelona (ES)**

(54) **VAGINAL RING FOR THE SIMULTANEOUS RELEASE OF TWO ACTIVE INGREDIENTS**

(57) It is provided a vaginal ring comprising a drug-loaded core comprising a progestogenic steroid compound, a estrogenic steroid compound, and an EVA copolymer having a vinyl acetate content from 25 to 35 wt.%, wherein the progestogenic steroid is dissolved in the core material in a relatively low degree of supersaturation; and a non-medicated outer layer of EVA copolymer having a vinyl acetate content from 5 to 15 wt.%; which is stable under storage at room temperature for at least 12 months. It is also provided a process for the preparation of the vaginal ring disclosed above.

EP 3 395 335 A1

**Description**

**Technical Field**

The present invention relates to the field of female contraception and hormone replacement therapy. Particularly, it relates to a drug delivery system for the simultaneous release of two active substances and more particularly to a ring shaped vaginal drug delivery system, which system releases the active substances in a substantially constant ratio over a prolonged period of time.

**Background Art**

Drug delivery systems, especially those intended for intravaginal use such as vaginal rings, are known in the art.
Release systems aimed at the release of two or more active substances in a substantially constant ratio to one another over a period of time are very useful for certain applications. For example, in the fields of contraception and hormone replacement therapy, extensive use is made of the simultaneous administration of an agent having a progestogenic activity and an agent having an estrogenic activity, preferably in a substantially constant ratio.
Among the possible polymeric materials used as reservoir and/or outer layer of the mentioned drugs, ethylene-vinylacetate copolymer (EVA copolymer) has resulted to provide the sought release ratio, while providing the rigidity as well as other mechanical properties required for the intended use.
WO 9702015 discloses a two-compartment device, a first compartment consisting of a core of EVA copolymer, a middle layer of EVA copolymer loaded with etonogestrel and a non-medicated outer layer of the same copolymer, and a second compartment consisting of loaded with both etonogestrel and ethinylestradiol, and a non-medicated outer layer of EVA copolymer. The preparation of the two component device requires cutting fibres into the desired lengths and the assembly of parts into the ring-shaped device.
As an improvement, EP 876815 discloses a vaginal ring (Nuvaring®) designed for the simultaneous release of a progestogenic steroid compound such as etonogestrel and a estrogenic steroid compound such as ethinylestradiol in the physiologically required fixed ratio over a prolonged period of time. The vaginal ring comprises a compartment comprising a thermoplastic polymer core containing a mixture of the progestogenic and the estrogenic compounds and an outer layer of thermoplastic polymer being permeable for the said progestogenic and estrogenic compounds. Said progestogenic compound is initially dissolved in the polymeric core material in a relatively low degree of supersaturation. Particularly, the progestogenic compound is initially dissolved in the core polymer in an amount from 1 to about 6 times of the amount by weight necessary for obtaining the saturation concentration of said progestogenic steroid in said core polymer at 25 °C, and the estrogenic compound is initially dissolved in the core polymer in a concentration being lower than that of the said progestogenic compound.
However, the commercialized product Nuvaring® (protected by EP 876815) is physically stable only under certain conditions. Particularly, prior to being dispensed to the user, it has to be stored refrigerated at 2-8 °C, and after being dispensed to the user, Nuvaring® can be stored for up to 4 months at a temperature below 30 °C. Thus, the product requires refrigeration both during storage and transport, which is expensive and requires a lot of attention. Particularly, if not stored below room temperature before being dispensed, the steroid can eventually crystallize on the outer surface of the vaginal ring, which may lead to an uncontrolled and sudden release.
It is, therefore, desirable to avoid the possibility of crystallization of the steroid on the outer surface of the vaginal ring when it is stored at or above room temperature (i.e. at a temperature from 20 to 30 °C, particularly at 25 °C). At the same time, the amount of steroid released and the release rate should remain unchanged to ensure a required pharmaceutical effect for use in contraception.

**Summary of Invention**

Inventors have found a new process that allows manufacturing a vaginal ring with a higher stability than the ones disclosed in the prior art. The new process allows obtaining a vaginal ring that remains stable at room temperature without any crystallization of any of the pharmaceutically active ingredients contained therein on the surface of the ring being produced.
Particularly, the vaginal ring of the invention is manufactured by an extrusion or coextrusion process similar to the one disclosed in EP 876815 but in some specific conditions. Unlike the vaginal ring disclosed in EP 876815, the vaginal ring of the invention remains physically stable when submitted to stability assays carried out at 30 °C/65% RH and 25 °C/60% RH for at least 12 months. As a consequence, the vaginal ring of the invention does not require any particular storage and/or transportation condition to preserve its properties. Particularly, it does not require to be kept at temperatures lower than 20 degrees, unlikely similar a vaginal ring known in the prior art requiring being stored refrigerated at 2-8 °C prior to being dispensed to the user.

Besides, stability assays under accelerated conditions (see Example 3) show that the vaginal ring of the invention is much more stable than a similar vaginal ring known in the prior art, Nuvaring®.

Accordingly, a first aspect of the present invention refers to a vaginal ring comprising:

- a drug-loaded core comprising a progestogenic steroid compound, a estrogenic steroid compound, and an EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and

- a non-medicated outer layer of EVA copolymer having a vinyl acetate content from 5 to 15 wt.%, particularly from 8 to 10 wt.%, more particularly of 9 wt.%; and

which is stable under storage at room temperature for at least 12 months,
wherein stability data are obtained according to ICH guidelines Q1A(R2) at 30 °C/65% RH, 25 °C/60% RH, and 5°C for at least 12 months.

A second aspect of the invention relates to a process for the manufacturing of the vaginal ring defined above, the process comprising:

a) extruding a mixture comprising a progestogenic steroid compound such as etonogestrel, a estrogenic steroid compound such as ethinylestradiol, and an ethylene-vinylacetate (EVA) copolymer having a vinyl acetate content from 25 to 35 wt.%, particularly from 27 to 29 wt.%, more particularly of 28 wt.%, in a extruder having an extrusion zone working with a temperature ramp;

b) cooling the material obtained in step a) to obtain a solid solution of the progestogenic steroid compound and the estrogenic steroid compound in the EVA copolymer;

c) coextruding the solid solution obtained in step b) with an EVA copolymer having a vinyl acetate content from 5 to 15 wt.%, particularly from 8 to 10 wt.%, more particularly of 9 wt.%, in order to obtain a fibre having:

- a drug-loaded core comprising the progestogenic steroid compound, the estrogenic steroid compound, and the EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and
- a non-medicated outer layer of EVA copolymer having a vinyl acetate content from 5 to 15 wt.%, particularly from 8 to 10 wt.%, more particularly of 9 wt.%; and

d) cutting the fibre obtained in step c) into pieces and joining the two ends of each peace to form a vaginal ring having a drug-loaded core and a non-medicated outer layer.

Brief Description of Drawings

Fig. 1 shows a planar cross-sectional view of a vaginal ring exemplifying the invention being a the core and b the outer layer.

Fig. 2 shows an axial cross section (i.e. cross-section along line A-B of Fig 1) of a vaginal ring exemplifying the invention, being a the core and b the outer layer.

Fig. 3a shows the X-ray diffraction pattern of a mixture of EVA copolymer having a 28 wt.% vinyl acetate content (EVA28), etonogestrel (0.637 wt.%) and ethinylestradiol (0.147 wt.%); 20 min of measurement time.

Fig. 3b shows the X-ray diffraction pattern of the EVA28, etonogestrel and ethinylestradiol mixture superimposed with the individual profiles of etonogestrel and ethinylestradiol (zoom at the 7-10 2theta range).

Fig. 4 shows the X-ray diffraction pattern of the etonogestrel ethinylestradiol vaginal ring of Example 1 compared with a Nuvaring®.

Fig. 5 shows the X-ray diffraction pattern of the etonogestrel ethinylestradiol vaginal ring of Example 1 compared with a Nuvaring® superimposed with the individual profiles of etonogestrel and ethinylestradiol (zoom at the 7-12 2theta range).

Fig. 6 and 7 show the increased release of ethinylestradiol and etonogestrel from the vaginal ring of Example 1 and Nuvaring®.

Fig. 8 and 9 show the *in vitro* release of ethinylestradiol and etonogestrel from a vaginal ring of Example 1 versus release from Nuvaring®.

Fig 10 and 11 show the *in vitro* release of ethinylestradiol and etonogestrel from the vaginal ring of Example 1 at 5 °C for 12 months.

Fig 12 and 13 show the *in vitro* release of ethinylestradiol and etonogestrel from the vaginal ring of Example 1 at 25 °C/60% RH for 12 months.

Fig 14 and 15 show the *in vitro* release of ethinylestradiol and etonogestrel from the vaginal ring of Example 1 at 30 °C/65% RH for 12 months.

Detailed description of the invention

As used herein, the term "coextrusion" refers to the extrusion of multiple layers of material simultaneously. This type of extrusion utilizes two or more extruders to melt and deliver a steady volumetric throughput of different viscous plastics to a single extrusion head (die) which will extrude the materials in the desired form.

As used herein, the term "solid solution" refers to a solid-state solution of one or more solutes in a solvent. Such a mixture is considered a solution rather than a compound when the crystal structure of the solvent remains unchanged by addition of the solutes, and when the mixture remains in a single homogeneous phase.

As used herein, the term "stable" and variations thereof refer to a state in which no crystallization of the steroid on the outer surface of the vaginal ring is produced when it is stored at room temperature (i.e. at a temperature from 20 to 30 °C, particularly at 25 °C). Said in other words, no significant migration of the active ingredients from the core of ethylene-vinylacetate copolymer containing 25 to 35 wt.%, particularly from 27 to 29 wt.%, more particularly of 28 wt.%, vinyl acetate to the outer layer of ethylene-vinylacetate copolymer containing 5 to 15 wt.%, particularly from 8 to 10 wt.%, more particularly of 9 wt.%, vinyl acetate is produced during storage at room temperature. At the same time, the amount of steroid released and the release rate remain unchanged, and thus the required pharmaceutical effect for use in contraception is ensured.

As mentioned above, a first aspect of the invention refers to a vaginal ring comprising a drug-loaded core comprising a progestogenic steroid compound, a estrogenic steroid compound, an EVA copolymer having a vinyl acetate content from 25 to 35 wt.%, particularly from 27 to 29 wt.%, more particularly of 28 wt.%, and, optionally, a lubricant such as magnesium stearate, stearic acid, and sodium stearyl fumarate, and wherein the progestogenic steroid is dissolved in the core material in a relatively low degree of supersaturation; and a non-medicated outer layer of EVA copolymer having a vinyl acetate content from 5 to 15 wt.%, particularly from 8 to 10 wt.%, more particularly of 9 wt.%. The outer layer of EVA copolymer is permeable for the said progestogenic and said estrogenic compounds. The vaginal ring is stable under storage at room temperature for at least 12 months.

As used herein, the term "for at least" a period of time, should be understood as "for a period equal to or higher than" the mentioned period.

In a particular embodiment, the drug-loaded core further comprises a lubricant such as magnesium stearate, stearic acid, and sodium stearyl fumarate. Particularly, the lubricant is magnesium stearate.

Particularly, the vaginal ring is obtainable by a process comprising mainly two stages:

The first stage is the extrusion of a mixture comprising a progestogenic steroidal compound such as etonogestrel, a estrogenic steroidal compound such as ethinylestradiol, and an EVA copolymer having a vinyl acetate content from 25 to 35 wt.%, particularly from 27 to 29 wt.%, more particularly of 28 wt.%, to obtain a solid solution of the two active ingredients in the copolymer, the extrusion being carried out in specific conditions. The extrusion mixture can also comprise a lubricant as defined above.

The second stage is the coextrusion of the abovementioned solid solution and an EVA copolymer having a vinyl acetate content from 5 to 15 wt.%, particularly from 8 to 10 wt.%, more particularly of 9 wt.%, in order to obtain a fibre having a drug-loaded core comprising the two active ingredients mentioned above in an EVA copolymer having a vinyl acetate content from 25 to 35 wt.%, particularly from 27 to 29 wt.%, more particularly of 28 wt.%, and a non-medicated outer layer of EVA copolymer having a vinyl acetate content from 5 to 15 wt.%, particularly from 8 to 10 wt.%, more particularly of 9 wt.%, wherein the non-medicated layer is the responsible of regulating the diffusion of the active ingredients.

The so obtained fibre is cut into pieces of the required length and the two ends of each peace are joined in any suitable manner, such as welding by heat contribution, to form a ring shaped device having a drug-loaded core and a non-medicated outer layer.

The rings are then packed, for example, in a suitable sachet, optionally after being sterilised and/or disinfected.

Accordingly, in a particular embodiment, the vaginal ring is obtainable by a process comprising:

a) extruding a mixture comprising a progestogenic steroid compound such as etonogestrel, a estrogenic steroid compound such as ethinylestradiol, and an ethylene-vinylacetate (EVA) copolymer having a vinyl acetate content from 25 to 35 wt.%, particularly from 27 to 29 wt.%, more particularly of 28 wt.%, in a extruder having an extrusion zone working with a temperature ramp up to 150 °C;

b) cooling the material obtained in step a) down to a temperature of from 30 to 50 °C to obtain a solid solution of the progestogenic steroid compound and the estrogenic steroid compound in the EVA copolymer;

c) coextruding the solid solution obtained in step b) with an EVA copolymer having a vinyl acetate content from 5 to 15 wt.%, particularly from 8 to 10 wt.%, more particularly of 9 wt.%, in order to obtain a fibre having:

- a drug-loaded core comprising the progestogenic steroid compound, the estrogenic steroid compound, and the EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and

- a non-medicated outer layer of EVA copolymer having a vinyl acetate content from 5 to 15 wt.%, particularly from 8 to 10 wt.%, more particularly of 9 wt.%; and

d) cutting the fibre obtained in step c) into pieces of the required length and joining the two ends of each peace in any suitable manner, such as welding by heat contribution, to form a vaginal ring having a drug-loaded core and a non-medicated outer layer.

In a particular embodiment, the mixture of step a) further comprises a lubricant such as magnesium stearate, stearic acid, and sodium stearyl fumarate which, accordingly, will form part of the drug-loaded core of the obtained vaginal ring.

Such an outer layer of an EVA copolymer having a vinyl acetate content from 8 to 10 wt.% has excellent solubility and steroid diffusion properties. This, in combination with the core comprising the two active ingredients in an EVA copolymer having a vinyl acetate content from 25 to 35 wt.%, particularly from 27 to 29 wt.%, more particularly of 28 wt.%, allows the combined release of the active ingredients, particularly of etonogestrel and ethinylestradiol, in the proper ratio at moderate concentrations of the steroids from the vaginal ring during a prolonged period of time. The percentage of vinyl acetate can be established using potentiometric titration as described in various textbooks on this subject matter.

As mentioned above the progestogenic steroid is dissolved in the core material in a relatively low degree of supersaturation. This "relatively low degree of supersaturation" may generally be defined as the amount of progestogenic steroid that is 1 to about 6 times the amount necessary to obtain the saturation concentration of the steroid in the polymer at 25 °C and particularly from 2 to 5 times. The saturation concentration of the steroid can be determined by various methods known per se in the art. For instance, the thermoplastic polymer can be introduced in a saturated solution of the steroid (provided with additional steroid crystals) at 25 °C and kept in that saturated solution until the concentration of the steroid in the polymer remains constant. Another suitable method for the determination of the saturation concentration is the so called time-lag method.

Accordingly, in a particular embodiment the progestogenic compound is initially dissolved in the core copolymer in an amount from 1 to 6 times, particularly from 2 to 5 times, of the amount by weight necessary for obtaining the saturation concentration of said progestogenic steroidal compound in said core polymer at 25 °C.

In another particular embodiment, optionally in combination with one or more features of the particular embodiments defined above, the estrogenic steroid compound is in a concentration lower than that of the progestogenic compound. Particularly, the ratio by weight of progestogenic steroidal compound and estrogenic steroidal compound is of 10 parts of the progestogenic steroidal compound and from 1.5-5 parts of the estrogenic steroidal compound.

The vaginal ring according to the invention is primarily designed for contraceptive use. Accordingly, in a particular embodiment the progestogenic steroidal compound is etonogestrel. In another particular embodiment the estrogenic steroidal compound is ethinylestradiol.

For contraception in humans, the vaginal ring according to the present invention is characterised by an EVA copolymer core comprising etonogestrel and ethinylestradiol in a ratio by weight of about 10 parts of etonogestrel and about 1.5-5 parts of ethinylestradiol, wherein etonogestrel is dissolved in the poly-EVA material up to a relatively low degree of supersaturation, particularly from 1 to 6 times its saturation concentration at 25 °C, so as to allow over a period of 21 days an average release rate of from 95 to 145 $\mu$g, particularly of 120 $\mu$g, of etonogestrel, and 10-20 $\mu$g, particularly 15 $\mu$g, of ethinylestradiol per 24 hours *in situ*.

A relatively low degree of supersaturation is obtained by using a quantity of etonogestrel in said poly-EVA core material of from 0.3 to 1 wt.%, the quantity of ethinylestradiol then being from 0.05 to 0.3 wt.%. In a more particular embodiment, the EVA copolymer core comprises from 0.5 to 1 wt.%, preferably from 0.55 to 0.8 wt.% of etonogestrel and from 0.10 to 0.23 wt.%, preferably from 0.12-0.18 wt.% by weight of ethinylestradiol.

The EVA copolymer has excellent mechanical and physical properties (e.g. solubility of the steroids in the material). The EVA copolymer material is used for both the core as well as the outer layer and can be any commercially available ethylene-vinylacetate copolymer, such as the products available under the trade names: Elvax, Evatane, Lupolen, Movriton, Ultrathene, Vestypar, Ateva, and Vitaldose.

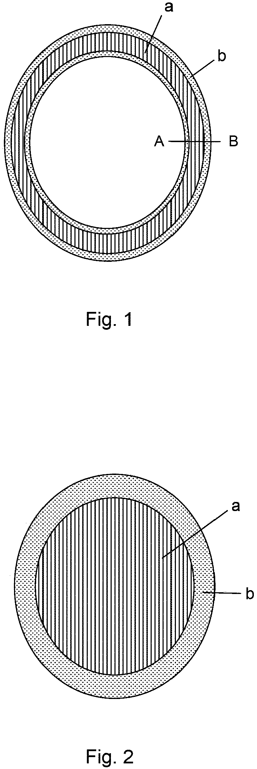

In a particular embodiment, optionally in combination with one or more features of the particular embodiments defined above or below, the vaginal ring has an outer diameter, i.e. a planar cross-sectional diameter, of from 52 to 56 mm, particularly of 54 mm. In a more particular embodiment, the vaginal ring has an axial cross sectional diameter (see Fig. 2) of from 2.5 to 5 mm, particularly of 4 mm.

In an even more particular embodiment, the core has a surface area of from 1700-2000 mm$^2$.

In a particular embodiment of the vaginal ring, the outer layer of the vaginal ring is formed by an EVA copolymer having a thickness from 100 to 120 $\mu$m.

As mentioned above, the vaginal ring obtainable by the process of the invention is particularly stable at room temperature, namely no crystallization on the surface of the ring of any of the pharmaceutically active ingredients is produced.

In order to obtain such a vaginal ring, the first step of the extrusion of the mixture of the progestogenic steroid compound, the estrogenic steroid compound, the ethylene-vinylacetate (EVA) copolymer having a vinyl acetate content from 25 to 35 wt.%, particularly from 27 to 29 wt.%, more particularly of 28 wt.%, and, optionally, a lubricant such as magnesium stearate, stearic acid, and sodium stearyl fumarate is carried out at a particular temperature ramp.

The thermal energy applied on the mixture, namely the temperature ramp in the extruder, is of high relevance. Thus, in a particular embodiment, optionally in combination with one or more features of the particular embodiments defined above, the extrusion zone comprises a loading zone, a pre-heating zone, a fusion zone, a blending zone, and an exit zone working at different temperatures.

In a more particular embodiment, optionally in combination with one or more features of the particular embodiments defined above, the temperature ramp is applied through the following 9 zones in the extruder: initial loading zone of the active mixture (T1), pre-heating zone (T2), fusion zone (T3), dispersing or blending zones (T5, T6), transporting zones (T4, T7), pressure zone (T8), and exit (T9) toward the cooling ramp.

The selection of these process parameters will affect not only on how the active ingredients go from a crystalline state to amorphous state to form a solid solution, but it will also influence the stabilization of the mentioned active ingredients in the matrix of the polymer and, therefore, the stability (storage conditions) and shelf life of the final vaginal ring.

The selected extrusion working temperatures are above the glass transition temperatures and melt temperatures of the ethylene-vinylacetate copolymer having a vinyl acetate content from 25 to 35 wt.%, particularly from 27 to 29 wt.%, more particularly of 28 wt.%, but well below the melt temperatures of the active ingredients of about 186 °C for ethinylestradiol and about 199 °C for etonogestrel. Thus, the polymer itself helps the dissolution of the active ingredients and decreases the probability that degradation products are produced.

In a particular embodiment, optionally in combination with one or more features of the particular embodiments defined above, the extrusion zone comprises 9 zones, wherein the temperature in each zone is the following:

T1) initial loading zone of the mixture of the progestogenic steroid compound, the estrogenic steroid compound, the EVA copolymer, and, optionally, a lubricant such as magnesium stearate, stearic acid, and sodium stearyl fumarate: from 45 to 55 °C, particularly from 47.5 to 52.5 °C, more particularly of 50 °C;

T2) pre-heating zone: from 105 to 135 °C, particularly from 110 to 130 °C, more particularly of 120 °C;

T3) fusion zone: from 135 to 165 °C, particularly from 140 to 160 °C, more particularly of 150 °C;

T4) transporting zone: from 65 to 115 °C, particularly from 75 to 105 °C, more particularly of 90 °C;

T5) dispersing or blending zone: from 65 to 95 °C, particularly from 70 to 90 °C, more particularly of 80 °C;

T6) dispersing or blending zone: from 55 to 85 °C, particularly from 60 to 80 °C, more particularly of 70 °C;

T7) transporting zone: from 55 to 85 °C, particularly from 60 to 80 °C, more particularly of 70 °C;
T8) pressure zone: from 55 to 85 °C, particularly from 60 to 80 °C, more particularly of 70 °C;
T9) exit towards the cooling ramp: from 70 to 100 °C, particularly from 75 to 95 °C, more particularly of 85 °C.

In another particular embodiment, optionally in combination with one or more features of the particular embodiments defined above, extrusion (step a)) is carried out at a constant feed rate and at a screw speed from 120 to 200 rpm, particularly from 130 to 190 rpm, more particularly of 160 rpm.

In another particular embodiment, optionally in combination with one or more features of the particular embodiments defined above, cooling of step b) is carried out by submitting the material obtained in step a) to a cooling ramp from 0.1 to 0.15 °C/cm of cooling conveyor belt.

In a particular embodiment, optionally in combination with one or more features of the particular embodiments defined above, the mixture in the extrusion zone (step a)) has a residence time of from 80 to 140 seconds, particularly of 110 seconds.

The specific mechanical energy consumption (SMEC) calculated for the product was 9215.4 kWh/kg $\pm$ 100 kWh/kg

The SMEC is calculated by the torque, the screw speed, and the feed rate, like shown in the following equation:

$$SMEC = \frac{\tau \cdot n}{\dot{m}} \left[ \frac{kJ}{kg} \right]$$

$\tau$: torque [Nm]; n: screw speed [rpm]; $\dot{m}$: throughput [kg/h].

This material is subsequently coextruded with temperatures once again higher than the glass transition temperatures and melting temperatures of both polymers (ethylene-vinylacetate copolymer having a vinyl acetate content from 5 to 15 wt.%, particularly from 8 to 10 wt.%, more particularly of 9 wt.%, and having a vinyl acetate content from 25 to 35 wt.%, particularly from 27 to 29 wt.%, more particularly of 28 wt.%) and inferior to the melting temperatures of the active ingredients.

In another particular embodiment, optionally in combination with one or more features of the particular embodiments defined above, coextrusion (step c)) is carried out with a first extruder and a second extruder which feed a coextrusion head, the first extruder supplying the solid solution obtained in step b), and the second extruder supplying the EVA copolymer having a vinyl acetate content from 8 to 10 wt.%, wherein the first extruder comprises four heating zones working at the following temperatures:

- Extruder 1: extrusion of a solid solution obtained in the first extrusion step comprising the two active ingredients and the ethylene vinylacetate copolymer having a vinyl acetate content from 25 to 35 wt.%, particularly from 27 to 29 wt.%, more particularly of 28 wt.%:

  Zone 1: from 50 to 80 °C, particularly from 55 to 75 °C, more particularly of 65 °C;
  Zone 2: from 60 to 90 °C, particularly from 65 to 85 °C, more particularly of 75 °C;
  Zone 3: from 70 to 100 °C, particularly from 75 to 95 °C, more particularly of 85 °C;
  Zone 4: from 70 to 100 °C, particularly from 75 to 95 °C, more particularly of 85 °C;

  wherein the second extruder comprises four heating zones working at the following temperatures:

- Extruder 2: extrusion of the outer layer of ethylene vinylacetate copolymer having a vinyl acetate content from 5 to 15 wt.%, particularly from 8 to 10 wt.%, more particularly of 9 wt.% (active diffusing regulator):

  Zone 1: from 100 to 130 °C, particularly from 105 to 125 °C, more particularly of 115 °C;
  Zone 2: from 135 to 165 °C, particularly from 140 to 160 °C, more particularly of 150 °C;
  Zone 3: from 150 to 180 °C, particularly from 155 to 175 °C, more particularly of 165 °C;
  Zone 4: from 140 to 210 °C, particularly from 150 to 200 °C, more particularly of 175 °C.

  and wherein the coextrusion head comprises 7 heating zones working at the following temperatures:

- Coextrusion head (in charge of applying the outer layer on the core layer):

  Zone 5 first extruder: from 75 to 105 °C, particularly from 80 to 100 °C, more particularly of 90 °C;

Zone 5 second extruder: from 160 to 190 °C, particularly from 165 to 185 °C, more particularly of 175 °C;
Zone 1 body: from 110 to 140 °C, particularly from 115 to 135 °C, more particularly of 125 °C;
Zone 2 body: from 110 to 140 °C, particularly from 115 to 135 °C, more particularly of 125 °C;
Zone 3 body: from 110 to 140 °C, particularly from 115 to 135 °C, more particularly of 125 °C;
Zone 4 body: from 110 to 140 °C, particularly from 115 to 135 °C, more particularly of 125 °C;
Zone 5 die: from 100 to 130 °C, particularly from 105 to 125 °C, more particularly of 115 °C.

As mentioned above, another aspect of the invention relates to a process as defined above for the manufacture of the vaginal ring defined above. All the particular embodiments disclosed above for the vaginal ring defined by its preparation process are also embodiments for the preparation process.

The invention also relates to the vaginal ring obtainable by the process as defined above.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

Examples

Example 1 - Preparation of a vaginal ring of the invention

103.4 g of etonogestrel (EG), 23.6 g of ethinylestradiol (EE), 15.0 g of magnesium stearate, and 14.8 kg of EVA copolymer having a 28 wt.% vinyl acetate content were mixed. This mixture was extruded at a constant feed rate of 3.0 kg/h and at a screw speed of 160 rpm. The extrusion zone of the extruder worked at the following temperatures:

T1) initial loading zone: 50 °C;
T2) pre-heating zone: 120 °C;
T3) fusion zone: 150 °C;
T4) transporting zone: 90 °C;
T5) dispersing or blending zone: 80 °C;
T6) dispersing or blending zone: 70 °C;
T7) transporting zone: 70 °C;
T8) pressure zone: 70 °C;
T9) exit towards the cooling ramp: 85 °C.

The extruded material obtained in step a) was subsequently submitted to a cooling ramp of 0.1-0.15 °C/cm of cooling conveyor belt. The obtained solid solution was coextruded with 1.74 kg of EVA copolymer having a 9 wt.% vinyl acetate content to form a co-axial fibre with an outer diameter of 4 mm, and an outer layer thickness of 110 $\mu$m. The fibre was cut into pieces of 157 mm. Subsequently, the ends of the fibre pieces were joined by welding (Fig. 1).

Example 2

The aim of this experiment was to detect if any crystalline APIs remaining in drug products just after their manufacturing process and a few weeks after their manufacturing process stored at the temperature pointed out below. The detection limit of the technique was estimated by measuring a mixture of the two APIs in the polymer matrix in powder form at the same concentration as the final drug product.

Determination of the crystalline/amorphous phase was performed by high power x-ray diffraction.

Samples measured and measurement conditions:

1. Physical mixture of EVA copolymer having a 28 wt.% vinyl acetate content, EG (0.637 wt.%) and EE (0.147 wt.%).
2. Nuvaring® A.V. (24 months, 5 °C) ref. 1147066
3. Vaginal ring of Example 1 (13 weeks, 5 °C) ref. SP000693
4. Vaginal ring of Example 1 (5 weeks, 5 °C) ref. SP001493

The drug product consisted on a cylinder (of about 4 mm diameter) that contained the abovementioned mixture in the inner part. The outer part was made of EVA copolymer having a 9 wt.% vinyl acetate content.

Powder diffraction data were collected at a wavelength of 0.95012Å in transmission geometry using the MYTHEN-II detector. Sample 1 (in powder form) was inserted in a glass capillary (1 mm diameter) without previous grinding. The measurements were performed in four different parts of the capillary (covering a total of 12 mm, which results in 9.4 mm$^3$ of sample) and merged afterwards. Short consecutive scans were done in each position to detect radiation damage if any. For the drug products (samples 2-4), slices of approx. 1 mm thickness were mounted in the diffractometer as free standing samples and measured with a small oscillation (in Chi and Phi) to increase statistics and probing volume.

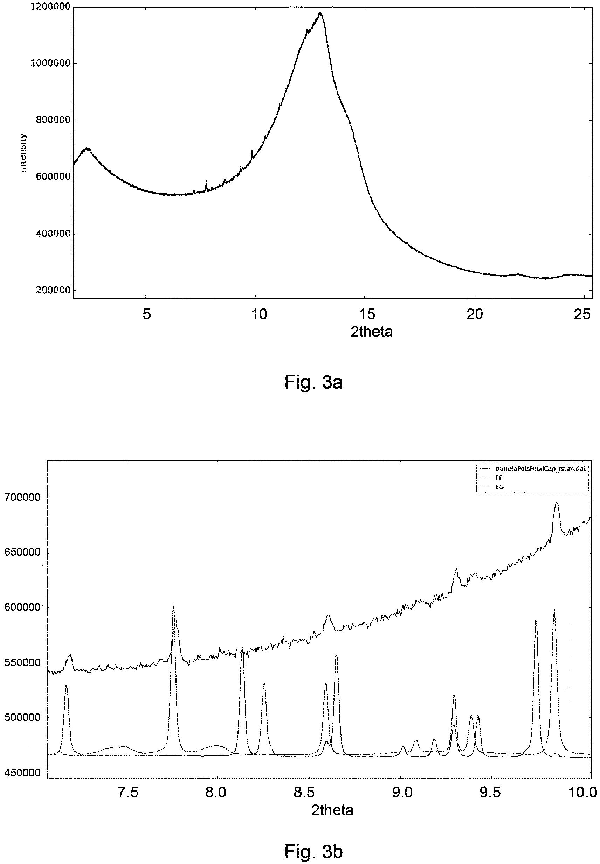

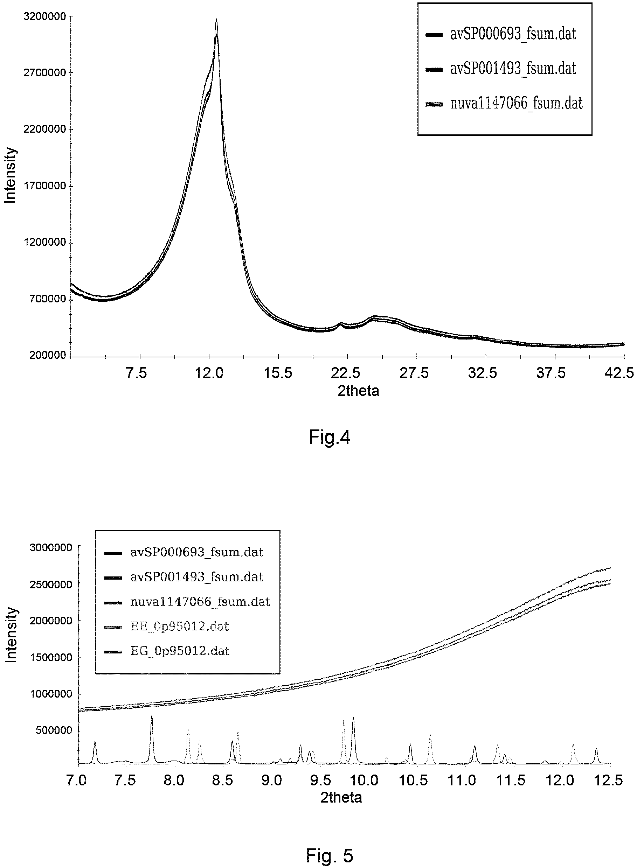

As shown in Fig. 4 in comparison with Fig. 3a and Fig. 3b, the results obtained confirmed the non-detection of crystalline phase in the samples of the vaginal rings of the invention and of the reference product, NuvaRing®. Thus, it was confirmed that both active ingredients are in amorphous form, namely in the form of a solid solution.

Example 3 - Migration studies

A Nuvaring® and a vaginal ring of Example 1 were subjected to a temperature of 70 °C for 1 month in order to accelerate the migration of the active ingredients from the core of ethylene-vinylacetate copolymer containing 28 wt.% vinyl acetate to the outer layer of ethylene-vinylacetate copolymer with a content of 9 wt.% vinyl acetate responsible for the release.

At the end of the month, a study of dissolution of the samples at the 4 h and 24h was carried out in order to evaluate the burst effect or dose dumping (very sudden and uncontrolled release of the active ingredients).

The dissolution conditions are detailed in the attached table:

| Device | USP 4 24411 |
| --- | --- |
| Release medium | Purified water |
| Volume | 200 ml |
| Temperature | 37 °C |

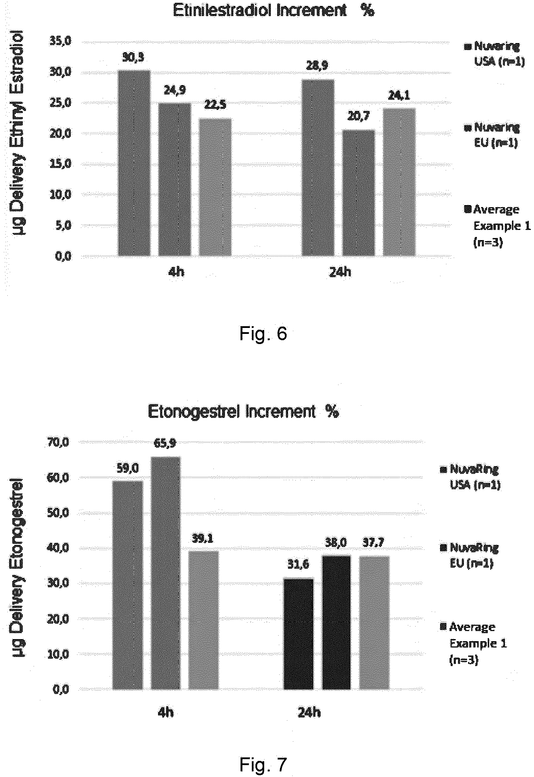

As shown in Fig. 6 and Fig. 7, the percentage incremental results confirmed that the impact of the temperature on the burst effect at 4 h is significantly lower for the vaginal ring of the invention than for Nuvaring® (both Nuvaring® product purchased in Europe and the one purchased in the USA).

Example 4 - Dissolution profile during 24 hours period

A comparative 24-hour dissolution profile study of 3 batches of the vaginal ring of Example 1 and a Nuvaring® batch was carried out. The conditions of the test were the described in example 3. In order to conduct a more intensive sampling in this period, one determination per hour was performed.

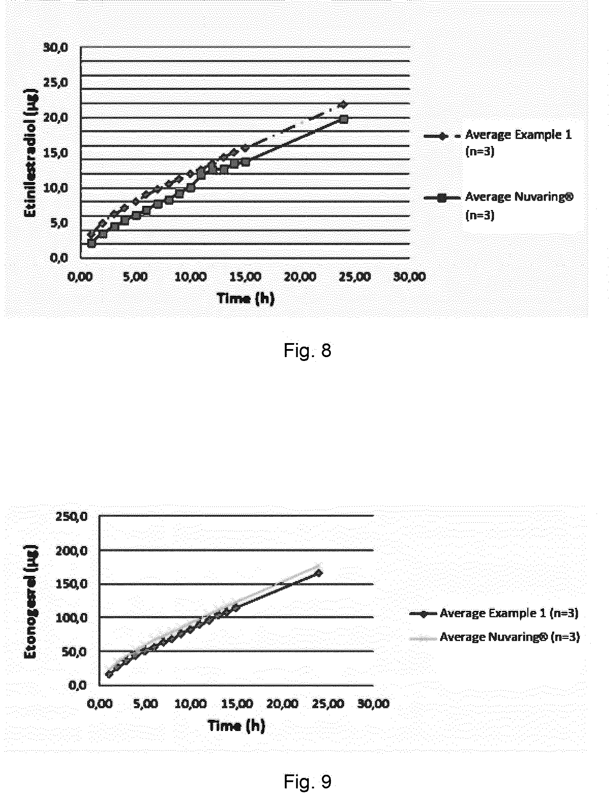

As shown in Fig. 8 and Fig. 9, the results confirmed that the dissolution rate of the vaginal ring of the invention during 24 hours is inferior than the one of Nuvaring®, what confirms the safety and the non-presence of a peak of dose dumping superior to Nuvaring®.

Example 5- ICH stability studies

ICH stability studies (ICH guidelines Q1A(R2) at 30 °C/65% RH, 25 °C/60% RH, and 5 °C) on the vaginal ring as obtained in Example 1 were carried out according to a dissolution test with the equipment and under the conditions shown below:

| Dissolution test | Ph. Eur. 2.9.3, 9th Edition 2017 |
| --- | --- |
| | HPLC liquid chromatography |
| | Reversed-phase method |
| | Detection of the UV region at 205 nm |
| | It is evaluated against an external standard |

| Equipment | 1. Dissolution equipment, flow-through apparatus |
| --- | --- |
| | 2. HPLC liquid chromatograph equipped with UV detector and computerized data processing system. |
| | 3. Ultrasonic bath |

(continued)

4. Mechanical stirrer

Dissolution bath conditions:

| | |
|---|---|
| Temperature | 37.0± 0.5° C (to reach 37.0°C put the bath to 38.5°C) |
| Volume | 200 mL |
| Flow rate | 16 mL/min |
| Pump speed | 120 rpm/min |
| Rotation speed (shaker) | 100 rpm |

Chromatographic conditions

| | |
|---|---|
| Column | X-Bridge C18, 4.6 x 250 mm and 5 $\mu$m |
| Mobile phase | Water/Acetonitrile (40:60) (v/v) |
| Flow | 1 mL/min |
| Column Temperature | 40°C |
| Detection | 205 nm ref off |
| Injection Volume | 100 $\mu$L |
| Chromatogram time | 10 min |

| | |
|---|---|
| Reagents | Purified water |

Acetonitrile

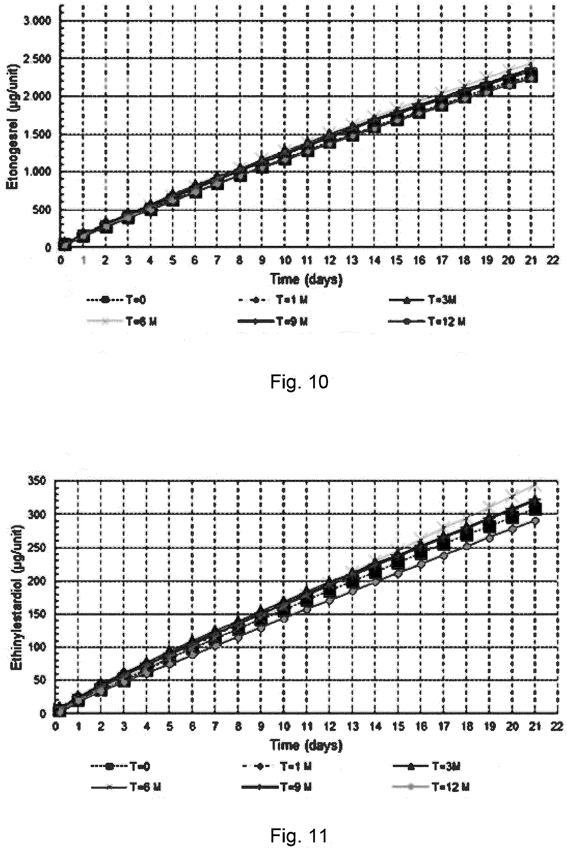

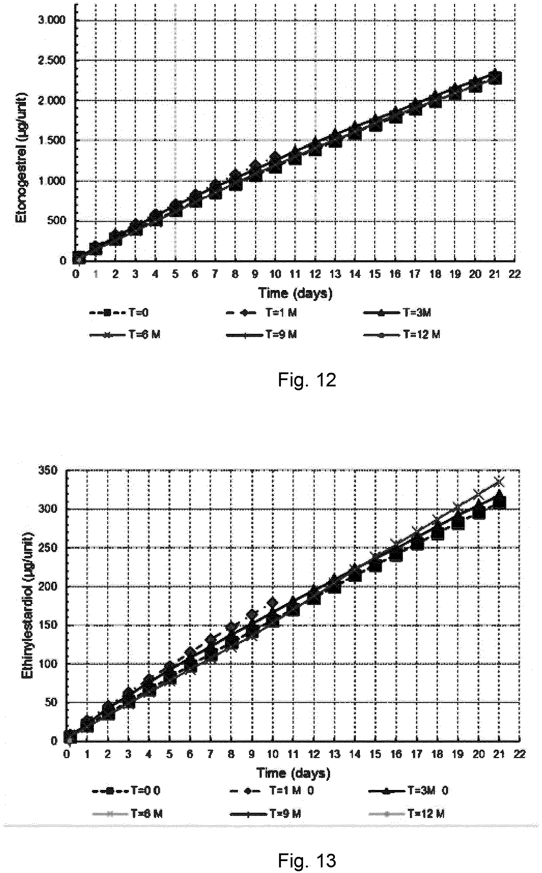

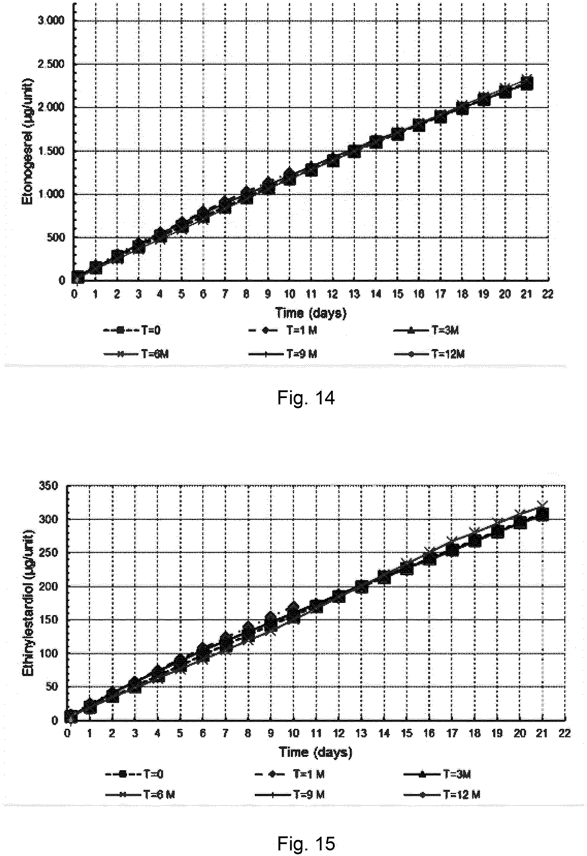

Results up to 12 months at 5 °C (real time conditions; see Fig. 10-11), up to 12 months at 25 °C/60% RH (intermediate conditions; see Fig. 12-13), and up to 12 months at 30 °C/65% RH (accelerated conditions; see Fig. 14-15) were obtained.

No trend indicating an impact of the preservation temperature on the *in vitro* release profile of the vaginal ring of Example 1 was observed. Conversely, as stated in the Nuvaring® Summary of Product Characteristics, the commercial product has to be stored refrigerated at 2-8 °C prior to being dispensed to the user, and can be stored for up to 4 months at a temperature below 30 °C after being dispensing.

**Citation List**

1. WO 9702015
2. EP 876815
3. Ph. Eur. 2.9.3, 9th Edition 2017

**Claims**

1.  A vaginal ring comprising:

    - a drug-loaded core comprising a progestogenic steroid compound, a estrogenic steroid compound, and an EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and
    - a non-medicated outer layer of EVA copolymer having a vinyl acetate content from 5 to 15 wt.%, particularly from 8 to 10 wt.%, more particularly of 9 wt.%; and

    which is stable under storage at room temperature for at least 12 months,
    wherein stability data are obtained according to ICH guidelines Q1A(R2) at 30 °C/65% RH, 25 °C/60% RH, and 5 °C for at least 12 months.

**2.** The vaginal ring according to claim 1, wherein the drug-loaded core further comprises a lubricant.

**3.** The vaginal ring according to claim 1, which is obtainable by a process comprising:

a) extruding a mixture comprising a progestogenic steroid compound, a estrogenic steroid compound, and an ethylene-vinylacetate (EVA) copolymer having a vinyl acetate content from 25 to 35 wt.%, particularly from 27 to 29 wt.%, more particularly of 28 wt.%, in a extruder having an extrusion zone working with a temperature ramp up to 150 °C;

b) cooling the material obtained in step a) down to a temperature of from 30 to 50 °C to obtain a solid solution of the progestogenic steroid compound and the estrogenic steroid compound in the EVA copolymer;

c) coextruding the solid solution obtained in step b) with an EVA copolymer having a vinyl acetate content from 5 to 15 wt.%, particularly from 8 to 10 wt.%, more particularly of 9 wt.%, in order to obtain a fibre having:

- a drug-loaded core comprising the progestogenic steroid compound, the estrogenic steroid compound, and the EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and

- a non-medicated outer layer of EVA copolymer having a vinyl acetate content from 5 to 15 wt.%, particularly from 8 to 10 wt.%, more particularly of 9 wt.%; and

d) cutting the fibre obtained in step c) into pieces and joining the two ends of each peace to form a vaginal ring having a drug-loaded core and a non-medicated outer layer.

**4.** The vaginal ring according to claim 3, wherein the extrusion zone comprises a loading zone, a pre-heating zone, a fusion zone, a blending zone, and an exit zone working at different temperatures.

**5.** The vaginal ring according to claim 3, wherein the extrusion zone comprises 9 zones, wherein the temperature in each zone is the following:

T1) initial loading zone of the mixture of the progestogenic steroid compound, the estrogenic steroid compound, and the EVA copolymer at a temperature from 45 to 55 °C;
T2) pre-heating zone at a temperature from from 105 to 135 °C;
T3) fusion zone at a temperature from 135 to 165 °C;
T4) transporting zone at a temperature from 65 to 115 °C;
T5) dispersing or blending zone at a temperature from 65 to 95 °C;
T6) dispersing or blending zone at a temperature from 55 to 85 °C;
T7) transporting zone at a temperature from 55 to 85 °C;
T8) pressure zone at a temperature from 55 to 85 °C;
T9) exit towards the cooling ramp at a temperature from 70 to 100 °C;

**6.** The vaginal ring according to any one of claims 3 to 5, wherein extrusion of step a) is carried out at a constant feed rate and at a screw speed from 120 to 200 rpm.

**7.** The vaginal ring according to any one of claims 3 to 6, wherein cooling of step b) is carried out by submitting the material obtained in step a) to a cooling ramp from 0.1 to 0.15 °C/cm of cooling conveyor belt.

**8.** The vaginal ring according to any one of claims 3 to 7, wherein the mixture in the extrusion zone has a residence time of from 80 to 140 seconds.

**9.** The vaginal ring according to any one of claims 3 to 8, wherein coextrusion step c) is carried out with a first extruder and a second extruder which feed a coextrusion head, the first extruder supplying the solid solution obtained in step b), and the second extruder supplying the EVA copolymer having a vinyl acetate content from 8 to 10 wt.%, wherein the first extruder comprises four heating zones working at the following temperatures:

Zone 1: from 50 to 80 °C,
Zone 2: from 60 to 90 °C,
Zone 3: from 70 to 100 °C,
Zone 4: from 70 to 100 °C,

wherein the second extruder comprises four heating zones working at the following temperatures:

Zone 1: from 100 to 130 °C,
Zone 2: from 135 to 165 °C,
Zona 3: from 150 to 180 °C,
Zona 4: from 140 to 210 °C,

and wherein the coextrusion head comprises 7 heating zones working at the following temperatures:

Zone 5 first extruder: from 75 to 105 °C,
Zone 5 second extruder: from 160 to 190 °C,
Zone 1 body: from 110 to 140 °C,
Zone 2 body: from 110 to 140 °C,
Zone 3 body: from 110 to 140 °C,
Zona 4 body: from 110 to 140 °C,
Zona 5 die: from 100 to 130 °C.

10. The vaginal ring according to any one of claims 1 to 9, wherein the progestogenic compound is in the core copolymer in an amount from 1 to 6 times of the amount by weight necessary for obtaining the saturation concentration of said progestogenic steroidal compound in said core polymer at 25 °C.

11. The vaginal ring according to any one of claims 1 to 10, wherein the estrogenic steroid compound is in the polymer core material in a concentration lower than that of the progestogenic compound.

12. The vaginal ring according to any one of claims 1 to 11, wherein the ratio by weight of progestogenic steroidal compound and the estrogenic steroidal compound is of 10 parts of the progestogenic steroidal compound and of from 1.5-5 parts of the estrogenic steroidal compound.

13. The vaginal ring according to any one of claims 1 to 12, wherein the progestogenic steroidal compound is etonogestrel, and the estrogenic steroidal compound is ethinylestradiol.

14. The vaginal ring according to any one of claims 1 to 13, wherein the EVA copolymer core comprises from 0.3 to 1 wt.% of etonogestrel and from 0.05 to 0.3 wt.% of ethinylestradiol.

15. A process for the manufacturing of a vaginal ring as defined in claim 1, the process comprising:

a) extruding a mixture comprising a progestogenic steroid compound , a estrogenic steroid compound, and an ethylene-vinylacetate (EVA) copolymer having a vinyl acetate content from 25 to 35 wt.%, particularly from 27 to 29 wt.%, more particularly of 28 wt.%, in a extruder having an extrusion zone working with a temperature ramp;
b) cooling the material obtained in step a) to obtain a solid solution of the progestogenic steroid compound and the estrogenic steroid compound in the EVA copolymer;
c) coextruding the solid solution obtained in step b) with an EVA copolymer having a vinyl acetate content from 5 to 15 wt.%, particularly from 8 to 10 wt.%, more particularly of 9 wt.%, in order to obtain a fibre having:

- a drug-loaded core comprising the progestogenic steroid compound, the estrogenic steroid compound, and the EVA copolymer having a vinyl acetate content from 25 to 35 wt.%, particularly from 27 to 29 wt.%, more particularly of 28 wt.%, wherein the progestogenic steroid is dissolved in the core material in a relatively low degree of supersaturation; and
- a non-medicated outer layer of EVA copolymer having a vinyl acetate content from 5 to 15 wt.%, particularly from 8 to 10 wt.%, more particularly of 9 wt.%; and

d) cutting the fibre obtained in step c) into pieces and joining the two ends of each peace to form a vaginal ring having a drug-loaded core and a non-medicated outer layer.

a

b

A —————— B

Fig. 1

a

b

Fig. 2

Fig. 3a

Fig. 3b

Fig.4

Fig. 5

## Etinilestradiol Increment %

Fig. 6

## Etonogestrel Increment %

Fig. 7

Fig. 8

Fig. 9

Fig. 10

Fig. 11

Fig. 12

Fig. 13

Fig. 14

Fig. 15

Europäisches
Patentamt

European
Patent Office

Office européen
des brevets

# EUROPEAN SEARCH REPORT

Application Number

EP 17 38 2218

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 732 520 A1 (ORGANON NV [NL]) 20 December 2006 (2006-12-20) | 1-15 | INV. A61K9/70 A61K31/565 |
| Y | * paragraph [0026] - paragraph [0027] * * paragraph [0039] * * paragraph [0051] - paragraph [0059] * * paragraph [0067] - paragraph [0070] * * tables 1-4 * * claims 1-4 * | 1-15 | |
| Y | US 2013/209539 A1 (LOXLEY ANDREW [US] ET AL) 15 August 2013 (2013-08-15) * example 1 * * claim 1 * | 1-15 | |
| Y | US 2015/157561 A1 (DE GRAAFF WOUTER [NL] ET AL) 11 June 2015 (2015-06-11) * example 1 * * claims 1-20 * | 1-15 | |
| X,D | EP 0 876 815 A1 (AKZO NOBEL NV [NL]) 11 November 1998 (1998-11-11) | 1,2, 10-14 | |
| Y | * page 3, line 42 - page 4, line 20 * * examples 1,2 * * claims 1-4 * | 1-15 | |

**TECHNICAL FIELDS SEARCHED (IPC)**

A61K

The present search report has been drawn up for all claims

| Place of search | Date of completion of the search | Examiner |
|---|---|---|
| Munich | 6 September 2017 | Sindel, Ulrike |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 17 38 2218

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2017

| Patent document cited in search report | | | Publication date | Patent family member(s) | | | Publication date |
|---|---|---|---|---|---|---|---|
| EP 1732520 | A1 | | 20-12-2006 | AR | 048185 | A1 | 05-04-2006 |
| | | | | AT | 434433 | T | 15-07-2009 |
| | | | | AU | 2005224056 | A1 | 29-09-2005 |
| | | | | BR | PI0509146 | A | 04-09-2007 |
| | | | | CA | 2559224 | A1 | 29-09-2005 |
| | | | | CN | 1933820 | A | 21-03-2007 |
| | | | | EP | 1732520 | A1 | 20-12-2006 |
| | | | | ES | 2327247 | T3 | 27-10-2009 |
| | | | | IL | 178018 | A | 17-05-2010 |
| | | | | JP | 2007530500 | A | 01-11-2007 |
| | | | | MY | 142824 | A | 14-01-2011 |
| | | | | PE | 01272006 | A1 | 15-03-2006 |
| | | | | TW | I290837 | B | 11-12-2007 |
| | | | | US | 2007141102 | A1 | 21-06-2007 |
| | | | | US | 2013266632 | A1 | 10-10-2013 |
| | | | | US | 2014302115 | A1 | 09-10-2014 |
| | | | | WO | 2005089723 | A1 | 29-09-2005 |
| US 2013209539 | A1 | | 15-08-2013 | AR | 089990 | A1 | 01-10-2014 |
| | | | | EP | 2814458 | A2 | 24-12-2014 |
| | | | | TW | 201332585 | A | 16-08-2013 |
| | | | | US | 2013209539 | A1 | 15-08-2013 |
| | | | | US | 2016000706 | A1 | 07-01-2016 |
| | | | | UY | 34625 | A | 02-09-2013 |
| | | | | WO | 2013120888 | A2 | 22-08-2013 |
| US 2015157561 | A1 | | 11-06-2015 | AU | 2014363724 | A1 | 26-05-2016 |
| | | | | CA | 2931559 | A1 | 18-06-2015 |
| | | | | CN | 105979930 | A | 28-09-2016 |
| | | | | CR | 20160261 | A | 16-08-2016 |
| | | | | DO | P2016000132 | A | 31-07-2016 |
| | | | | EA | 201691193 | A1 | 30-09-2016 |
| | | | | EP | 3079660 | A1 | 19-10-2016 |
| | | | | HK | 1224208 | A1 | 18-08-2017 |
| | | | | JP | 2016539991 | A | 22-12-2016 |
| | | | | KR | 20160095110 | A | 10-08-2016 |
| | | | | MD | 20160079 | A2 | 31-10-2016 |
| | | | | PE | 06802016 | A1 | 31-07-2016 |
| | | | | PH | 12016501083 | A1 | 25-07-2016 |
| | | | | SG | 11201604660V | A | 28-07-2016 |
| | | | | SV | 2016005214 | A | 21-11-2016 |
| | | | | TW | 201607563 | A | 01-03-2016 |
| | | | | US | 2015157561 | A1 | 11-06-2015 |
| | | | | WO | 2015086491 | A1 | 18-06-2015 |
| EP 0876815 | A1 | | 11-11-1998 | AR | 011709 | A1 | 30-08-2000 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 17 38 2218

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2017

| Patent document cited in search report | Publication date | Patent family member(s) | | Publication date |
|---|---|---|---|---|
| | | AT | 211646 T | 15-01-2002 |
| | | AU | 726934 B2 | 23-11-2000 |
| | | BR | 9801027 A | 11-01-2000 |
| | | CA | 2233753 A1 | 11-10-1998 |
| | | CN | 1196930 A | 28-10-1998 |
| | | CZ | 9801102 A3 | 14-10-1998 |
| | | DE | 69803112 D1 | 14-02-2002 |
| | | DE | 69803112 T2 | 18-07-2002 |
| | | DK | 0876815 T3 | 22-04-2002 |
| | | EP | 0876815 A1 | 11-11-1998 |
| | | ES | 2171283 T3 | 01-09-2002 |
| | | HK | 1016886 A1 | 03-05-2002 |
| | | HU | 9800863 A2 | 01-02-1999 |
| | | ID | 21259 A | 12-05-1999 |
| | | IL | 123813 A | 25-11-2001 |
| | | JP | 4982670 B2 | 25-07-2012 |
| | | JP | H10287549 A | 27-10-1998 |
| | | JP | 2009256378 A | 05-11-2009 |
| | | NO | 981638 A | 12-10-1998 |
| | | NZ | 330145 A | 28-05-1999 |
| | | PL | 325774 A1 | 12-10-1998 |
| | | PT | 876815 E | 31-07-2002 |
| | | RU | 2206325 C2 | 20-06-2003 |
| | | SG | 65061 A1 | 25-05-1999 |
| | | TR | 9800648 A1 | 21-10-1998 |
| | | TW | 358031 B | 11-05-1999 |
| | | US | 5989581 A | 23-11-1999 |
| | | ZA | 9802911 B | 09-10-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9702015 A

- EP 876815 A

**Non-patent literature cited in the description**

- Ph. Eur. 2.9.3. 2017